(19) Europäisches Patentamt
European Patent Office
Office européen des brevets

(11) Veröffentlichungsnummer: **0 319 835 B1**

# (12) EUROPÄISCHE PATENTSCHRIFT

(45) Veröffentlichungstag der Patentschrift: **10.03.93**

(51) Int. Cl.[5]: **C07C 249/04**, C07C 251/32, C07C 259/00, C07C 323/00, C07D 333/28, C07D 325/00, C07D 327/00, C07D 339/00, A01N 35/10, A01N 43/02

(21) Anmeldenummer: **88119922.8**

(22) Anmeldetag: **30.11.88**

Die Akte enthält technische Angaben, die nach dem Eingang der Anmeldung eingereicht wurden und die nicht in dieser Patentschrift enthalten sind.

## (54) Cyclohexenonverbindungen, Verfahren zu ihrer Herstellung und ihre Verwendung zur Bekämpfung unerwünschten Pflanzenwachstums.

(30) Priorität: **10.12.87 DE 3741823**

(43) Veröffentlichungstag der Anmeldung:
**14.06.89 Patentblatt 89/24**

(45) Bekanntmachung des Hinweises auf die Patenterteilung:
**10.03.93 Patentblatt 93/10**

(84) Benannte Vertragsstaaten:
**AT BE CH DE FR GB IT LI NL**

(56) Entgegenhaltungen:
**EP-A- 0 133 349**
**EP-A- 0 137 174**
**AU-B- 11 117**
**GB-A- 2 137 200**

(73) Patentinhaber: **BASF Aktiengesellschaft**
**Carl-Bosch-Strasse 38**
**W-6700 Ludwigshafen(DE)**

(72) Erfinder: **Keil, Michael, Dr.**
**Fontanestrasse 4**
**W-6713 Freinsheim(DE)**
Erfinder: **Schirmer, Ulrich, Dr.**
**Berghalde 79**
**W-6900 Heidelberg(DE)**
Erfinder: **Kolassa, Dieter, Dr.**
**Moltkestrasse 8**
**W-6700 Ludwigshafen(DE)**
Erfinder: **Kast, Jürgen, Dr.**
**Kastanienstrasse 24**
**W-6737 Boehl-Iggelheim(DE)**
Erfinder: **Würzer, Bruno, Dr.**
**Rüdigerstrasse 13**
**W-6701 Otterstadt(DE)**
Erfinder: **Meyer, Norbert, Dr.**
**Dossenheimer Weg 22**
**W-6802 Ladenburg(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.5x/3.0.1)

## Beschreibung

Die Erfindung betrifft Cyclohexenonverbindungen, Verfahren zu ihrer Herstellung, sowie Herbizide, die diese Verbindungen als Wirkstoffe enthalten.

Es ist bekannt, Cyclohexenonverbindungen zur Bekämpfung von unerwünschten Gräsern in breitblättrigen Kulturen zu verwenden (DE-OS 24 39 104). Außerdem ist aus DE-OS 32 48 554 und DE-OS 33 29 017 bekannt, daß Cyclohexen-1-onverbindungen, die in 5-Stellung einen para-substituierten Phenylrest tragen, grasartige Unkräuter in Weizen bekämpfen.

Ferner werden in der GB-A-2 137 200 5-Phenyl-cyclohexan-1,3-dione vom Typ der Verbindungen I beschrieben, für die u.a. eine herbizide Wirkung angegeben wird. Im Gegensatz zu den erfindungsgemäßen Verbindungen I trägt der Phenyl-Substituent am Cyclohexenon-Grundkörper jedoch mindestens einen Rest $-CH_2-OR'$, $-CH_2-SR'$ oder $-CH_2-N(R')_2$, wobei R' Wasserstoff, Alkyl, Alkenyl, Alkinyl, Cycloalkyl oder gegebenenfalls substituiertes Benzyl bedeutet.

Es wurde nun gefunden, daß Cyclohexenonverbindungen der Formel I

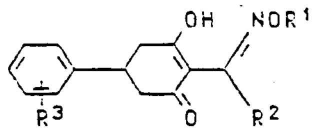

(I)

in der die Substituenten folgende Bedeutung haben:

- $R^1$ Alkyl mit 1 bis 4 C-Atomen, Alkenyl oder Alkinyl mit 3 oder 4 C-Atomen, Haloalkenyl mit 3 oder 4 C-Atomen und 1 bis 3 Halogensubstituenten, ggf. bis zu dreifach halo- und/oder $C_1$-$C_4$-alkylsubstituiertes Thenyl,
- $R^2$ Alkyl mit 1 bis 4 C-Atomen und
- $R^3$ Formyl, ein Rest der allgemeinen Formel $R^4XCHXR^5$, wobei X = O oder S und $R^4$, $R^5$ gleiches oder verschiedenes Alkyl oder gemeinsam Alkylen mit jeweils 1 bis 4 C-Atomen bedeutet und der zusätzlich durch $C_1$-$C_4$-Alkyl, $C_1$-$C_4$-Alkoxy, $C_1$-$C_4$-Alkylthio, Hydroxy, Halogen, Cyano, $C_1$-$C_4$-N,N-Dialkylamino substituiert sein kann,

und ihre biologisch annehmbaren Salze mit Alkalimetall-, Erdalkalimetall- und Ammoniumionen sowie ihre Ester mit $C_1$-$C_{10}$-Carbonsäuren und anorganischen Säuren, eine gute herbizide Wirkung vorzugsweise gegen Arten aus der Familie der Gräser (Gramineen) besitzen. Sie sind verträglich und somit selektiv in breitblättrigen Kulturen sowie in monokotylen Gewächsen, welche nicht zu den Gramineen zählen. Einige Verbindungen verhalten sich auch in Gramineenkulturen wie z.B. Weizen, Gerste oder Reis selektiv in dem Sinne, daß sie unerwünschte Gräser bekämpfen, ohne die Nutzpflanzen nennenswert zu schädigen.

Die Formel I gibt nur eine der möglichen tautomeren Formen wieder; es sind mehrere andere Formen möglich, die, wie die nachstehende Darstellung zeigt

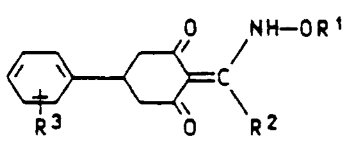

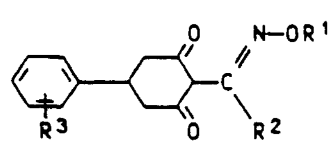

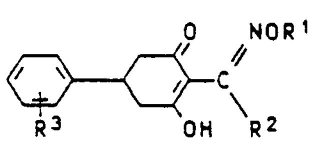

bzw.

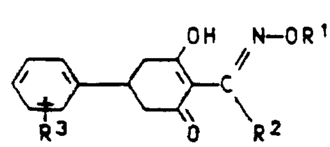

sämtlich als Glieder der allgemeinen Formel I gelten sollen.

Die erfindungsgemäßen Cyclohexenonverbindungen sind offensichtlich Säuren, d.h. sie können einfache Umsetzungsprodukte wie Salze von Alkali- oder Erdalkaliverbindungen oder Ester bilden. Die entsprechenden Salze oder Ester gehören mit zur Erfindung.

$R^1$ in Formel I bedeutet beispielsweise Ethyl, n-Propyl, Allyl, vorzugsweise Chloralkenyl wie (E)-3-Chlor-2-propenyl, 2-Chlor-2-propenyl oder 2,3,3-Trichlor-2-propenyl und weiter z.B. Propargyl, 2-Butinyl, (E)-2-Butenyl und ferner 5-Chlor-2-thenyl.

$R^2$ in Formel I steht für Methyl, Ethyl, n-Propyl, i-Propyl, n-Butyl, sec.-Butyl, i-Butyl und tert.-Butyl.

$R^3$ in Formel I steht für Formyl.

Dialkoxymethyl mit 3 bis 9 Kohlenstoffatomen, beispielsweise Dimethoxymethyl, Diethoxymethyl, Dipropoxymethyl, Dibutoxymethyl, Di(2-methoxyethoxy)methyl, Di(2-ethylthioethoxy)methyl, Di(2-chlorethoxy)-methyl, Di(2,2,2-trichlorethoxy)methyl, Di(3-brompropoxy)methyl, Di(2,2,3,3,4,4,4-heptafluorbutoxy)methyl, Di(3-methoxy-1-methyl-propoxy)methyl,

Dialkylthiomethyl mit 3 bis 9 Kohlenstoffatomen, beispielsweise Di(methylthio)methyl, Di(ethylthio)-methyl, Di(propylthio)methyl, Di(butylthio)methyl, Di(2-ethoxyethylthio)methyl, Di(2-ethylthioethylthio)methyl, Di(3-chlorpropylthio)methyl, Di(2-cyanoethylthio)methyl, Di(2-N,N-dimethylaminoethylthio)methyl.

Dioxolanyl mit 3 bis 9 Kohlenstoffatomen, beispielsweise 4-Ethyl-1,3-dioxolan-2-yl, 4,5-Dimethyl-1,3-dioxolan-2-yl, 4,4-Dimethyl-1,3-dioxolan-2-yl, 4,4,5-Trimethyl-1,3-dioxolan-2-yl, 4-Chlormethyl-1,3-dioxolan-2-yl,

4-Methylthioethyl-1,3-dioxolan-2-yl, 4-Hydroxymethyl-1,3-dioxolan-2-yl, Dithiolanyl mit 3 bis 9 Kohlenstoffatomen, beispielsweise 1,3-Dithiolan-2-yl, 4-Methyl-1,3-dithiolan-2-yl,

Oxothiolanyl mit 3 bis 9 Kohlenstoffatomen, beispielsweise 1,3-Oxothiolan-2-yl,

Dioxanyl mit 3 bis 9 Kohlenstoffatomen, beispielsweise 4-Methyl-1,3-dioxan-2-yl, 5,5-Dimethyl-1,3-dioxan-2-yl, 4,4-Dimethyl-1,3-dioxan-2-yl, 4,4,6-Trimethyl-1,3-dioxan-2-yl, 5-Methoxymethyl-5-methyl-1,3-dioxan-2-yl und

Dithianyl mit 3 bis 9 Kohlenstoffatomen, beispielsweise 1,3-Dithian-2-yl.

Die Oximether der Formel Ib erhält man aus den Acylderivaten der Formel II in einem zweistufigen Prozeß. Der erste Schritt beinhaltet die chemoselektive Bildung des Acetals oder Thioacetals, das nach literaturbekannten Verfahren - eine Übersicht findet sich in C. Ferri "Reaktionen der organischen Synthese" S. 426ff und S. 491 (1978) - erhalten wird. Im zweiten Schritt wird dieses Acetal/Thioacetal mit einem entsprechenden Hydroxylaminsalz $R^1ONH_3Y$, wobei Y ein beliebiges Anion bedeutet, zum Oximether der Formel Ib umgesetzt.

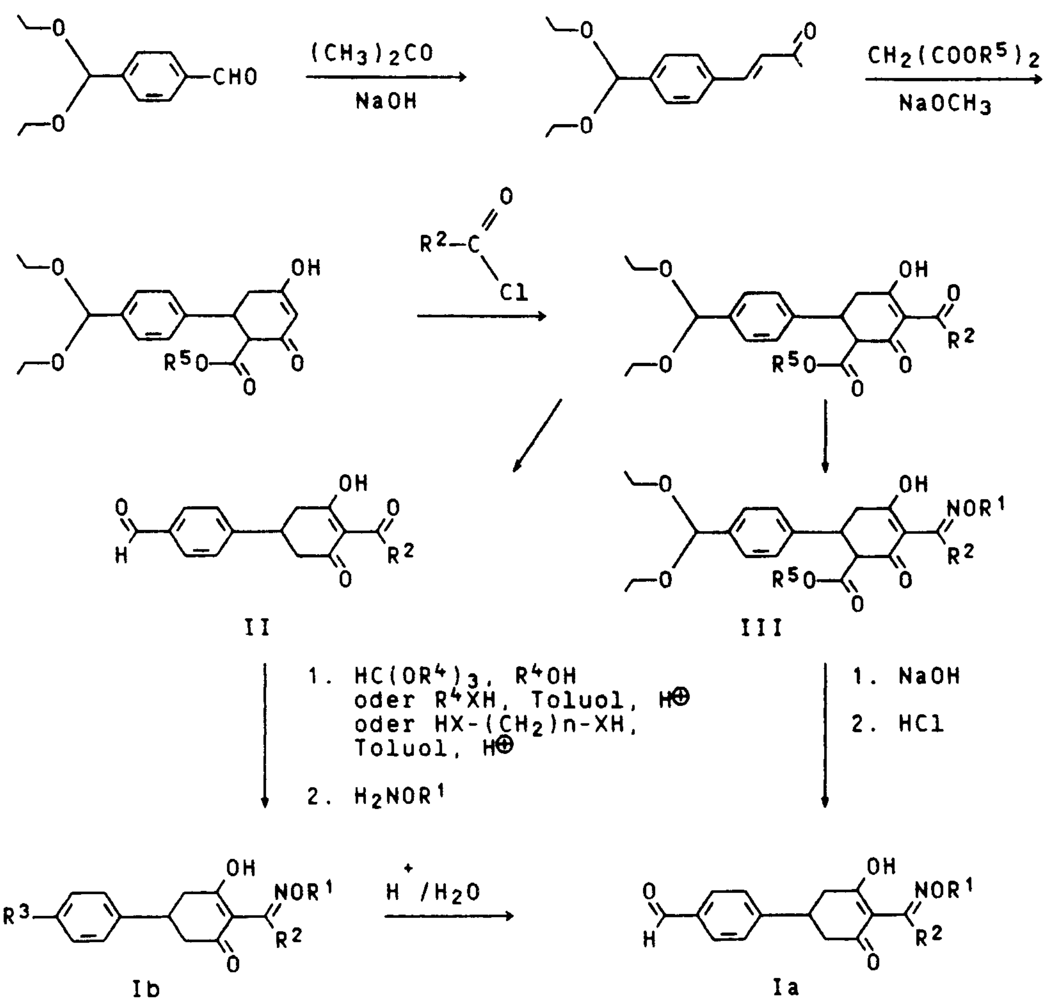


II

III

Ib

Ia

Man führt diese Umsetzung zweckmäßigerweise in heterogener Phase in einem Verdünnungsmittel bei einer ausreichenden Temperatur oberhalb von 0 °C und unterhalb des Siedepunktes des Reaktionsgemisches in Gegenwart einer Base durch. Geeignete Basen sind beispielsweise Carbonate, Hydrogencarbonate, Acetate, Alkoholate, Hydroxide oder Oxide von Alkali- oder Erdalkalimetallen, insbesondere von Natrium, Kalium, Magnesium, Calcium. Außerdem können auch organische Basen wie Pyridin oder tertiäre Amine Verwendung finden.

Die Umsetzung verläuft besonders gut in einem pH-Bereich von 2 bis 9, insbesondere von 4,5 bis 5,5. Die Einstellung des pH-Bereiches erfolgt zweckmäßigerweise durch Zusatz eines puffernden Acetats, beispielweise von Natrium- oder Kaliumacetat oder einer Mischung aus beiden Salzen. Alkalimetallacetate werden beispielsweise in Mengen von 0,5 bis 2 Mol, bezogen auf die Ammoniumverbindung der Formel $R^{1}0\text{-}NH_3Y$, zugesetzt.

Als Lösungsmittel sind beispielsweise Dimethylsulfoxid, Alkohole, wie Methanol, Ethanol, Isopropanol, Benzol, gegebenenfalls chlorierte Kohlenwasserstoffe, wie Chloroform, Dichlorethan, Hexan, Cyclohexan, Ester, wie Essigsäureethylester, Ether, wie Dioxan, Tetrahydrofuran, geeignet.

Die Umsetzung ist nach wenigen Stunden beendet, das Reaktionsprodukt kann dann durch Einengen der Mischung, Zugabe von Wasser und Extraktion mit einem unpolaren Lösungsmittel wie Methylenchlorid und Abdestillieren des Lösungsmittels unter vermindertem Druck isoliert werden.

Verbindungen der Formel I können außerdem durch Umsetzen von entsprechenden Verbindungen der Formel II mit dem entsprechenden freien Hydroxylamin $R^{1}0\text{-}NH_2$ in einem Verdünnungsmittel bei einer Temperatur zwischen 0 °C und dem Siedepunkt des Reaktionsgemisches, insbesondere zwischen 15 und 70 °C erhalten werden. Gegebenenfalls kann das Hydroxylamin als wäßrige Lösung eingesetzt werden.

Geeignete Lösungsmittel für diese Umsetzung sind beispielsweise Alkohole, wie Methanol, Ethanol, Isopropanol, Cyclohexanol, gegebenenfalls chlorierte Kohlenwasserstoffe, wie Hexan, Cyclohexan, Methylenchlorid, Toluol, Dichlorethan, Ester, wie Essigsäureethylester, Nitrile, wie Acetonitril, cyclische Ether, wie Tetrahydrofuran.

Die Verbindungen der Formel Ia mit $R^3$ = Formyl erhält man beispielsweise durch Verseifung der Acetalester III ($R^3$ = Dialkoxymethyl) mittels einer wäßrigen Alkalihydroxydlösung, beispielsweise Natronlauge oder Kalilauge, bei einer Temperatur zwischen 0 bis 100 °C, vorzugsweise Raumtemperatur. Beim anschließenden Ansäuern auf pH 1 bis 5 mit einer gängigen Säure wie beispielsweise Salzsäure, Schwefelsäure, Phosphorsäure, Essigsäure, Oxalsäure, Ameisensäure wird unter gleichzeitiger Decarboxylierung und Acetalspaltung der gewünschte Aldehyd gebildet und ggf. durch Extraktion isoliert. Als Extraktionsmittel können hierbei alle nicht wassermischbaren organischen Lösungsmittel dienen, beispielsweise Toluol, Dichlormethan, Essigester.

Weiterhin können Verbindungen der Formel Ia durch Spaltung der Acetale und Thioacetale der Formel Ib erhalten werden. In Th. W. Greene "Protective Groups in Organic Synthesis", S. 115-138 (1981) sind die üblichen Verfahren ausführlich dokumentiert.

Beispiel 1

5-(4-Dimethoxymethylphenyl)-2-(1-ethoxyiminobutyl)-3-hydroxycyclohex-2-en-1-on

8,4 g 2-Butyryl-5-(4-dimethoxymethylphenyl)-3-hydroxycyclohex-2-en-1-on werden mit 2,5 g Ethoxyamin, 2,1 g Natriumhydrogencarbonat und 100 ml Methanol versetzt und 10 Stunden bei Raumtemperatur gerührt. Nach Einengen wurde der Rückstand in Methylenchlorid/Wasser verteilt, die organische Phase abgetrennt, getrocknet und eingeengt. Es verbleiben 7,5 g 5-(4-Dimethoxymethylphenyl)-2-(1-ethoxyiminobutyl)-3-hydroxycyclohex-2-en-1-on (Verbindung Nr. 19 der nachstehenden Tabelle):

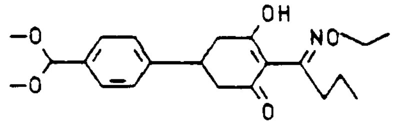

Beispiel 2

2-(1-Ethoxyiminobutyl)-5-(4-formylphenyl)-3-hydroxycyclohex-2-en-1-on

2,0 g der Verbindung Nr. 19 (Beispiel 1) wurden mit einem Gemisch aus 3,5 g Kieselgel (70 - 270 mesh), 5 ml Dichlormethan und 0,35 g 10 %iger Oxalsäure 15 Stunden bei Raumtemperatur behandelt. Die Mischung wurde abfiltriert, mit Dichlormethan nachgewaschen und das Filtrat eingeengt. Es verblieben 1,5 g 2-(1-Ethoxyiminobutyl)-5-(4-formylphenyl)-3-hydroxycyclohex-2-en-1-on als Feststoff vom Smp. 81 - 82 °C (Verb. Nr. 8).

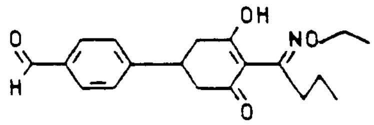

Die in der nachstehenden Tabelle aufgeführten Verbindungen können aus geeigneten Vorprodukten evtl. nach geringfügiger Anpassung der vorstehenden Beispielsangaben hergestellt werden; sie lassen eine vergleichbare Wirkung erwarten. Eine repräsentative Auswahl von Verbindungen (gekennzeichnet durch physikalische Angaben) wurde hergestellt und auf biologische Wirkung untersucht.

Tabelle

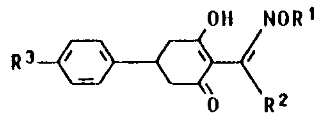

(I)

| Wirkstoff Nr. | $R^3$ | $R^2$ | $R^1$ | Smp. [°C] bzw. charakterist. $^1$H-NMR-Daten [δ ppm] |
|---|---|---|---|---|
| 1 | CHO | $C_2H_5$ | $C_2H_5$ | 1,17(t), 1,33(t), 2,97(q), 4,13(q), 10,0(s) |
| 2 | CHO | $C_2H_5$ | n-$C_3H_7$ | 1,0(t), 2,96(t), 3,10(q), 4,03(t), 10,0(s) |
| 3 | CHO | $C_2H_5$ | $CH_2$-CH=$CH_2$ | 1,18(t), 2,97(t), 4,58(d), 10,01(s) |
| 4 | CHO | $C_2H_5$ | (E)-$CH_2$-CH=CHCl | 1,17(t), 2,92(q), 4,55(d), 6,12(m), 6,36(d), 10,0(s) |
| 5 | CHO | $C_2H_5$ | (E)-$CH_2$-CH=CH-$CH_3$ | 1,18(t), 1,78(d), 2,97(q), 4,47(d), 10,0(s) |
| 6 | CHO | $C_2H_5$ | $CH_2$-C≡CH | 1,16(t), 2,90(q), 4,67(d), 10,01(s) 114-16 |
| 7 | CHO | $C_2H_5$ | $CH_2$-C≡C-$CH_3$ | |
| 8 | CHO | n-$C_3H_7$ | $C_2H_5$ | 81 - 82 |
| 9 | CHO | n-$C_3H_7$ | n-$C_3H_7$ | 0,99(t);4,03(t);10,01(s) 66-68 |
| 10 | CHO | n-$C_3H_7$ | $CH_2$-CH=$CH_2$ | 1,01(t);5,96(m);10,01(s) 63-64 |
| 11 | CHO | n-$C_3H_7$ | (E)-$CH_2$-CH=CHCl | 0,99(t);6,11(m);6,36(d) |
| 12 | CHO | n-$C_3H_7$ | (E)-$CH_2$-CH=CH-$CH_3$ | |
| 13 | CHO | n-$C_3H_7$ | $CH_2$-C≡CH | 0,98(t), 2,65(s), 4,67(d), 10,01(s) 72-73 |
| 14 | CHO | n-$C_3H_7$ | $CH_2$-C≡C-$CH_3$ | 68-70 |
| 15 | $CH(OCH_3)_2$ | $C_2H_5$ | $C_2H_5$ | 1,19(t), 1,33(t), 2,97(q), 3,34(s), 4,15(q), 5,37(s) |

Tabelle (Fortsetzung)

| Wirkstoff Nr. | $R^3$ | $R^2$ | $R^1$ | Smp. [°C] bzw. charakterist. $^1H$-NMR-Daten [δ ppm] |
|---|---|---|---|---|
| 16 | $CH(OCH_3)_2$ | $C_2H_5$ | $CH_2-CH=CH_2$ | 1,18(t), 2,95(q), 3,32(s), 4,55(d), 5,36(dd) |
| 17 | $CH(OCH_3)_2$ | $C_2H_5$ | (E)-$CH_2-CH=CHCl$ | 1,16(t), 2,91(q), 3,33(s), 4,53(d), 5,39(s) |
| 18 | $CH(OCH_3)_2$ | $C_2H_5$ | (E)-$CH_2-CH=CH-CH_3$ | 1,17(t), 1,77(d), 2,96(q), 3,33(s), 4,48(d), 5,38(s) |
| 19 | $CH(OCH_3)_2$ | n-$C_3H_7$ | $C_2H_5$ | 0,98(t);3,33(s);4,10(s) |
| 20 | $CH(OCH_3)_2$ | n-$C_3H_7$ | $CH_2-CH=CH_2$ | 0,99(t), 2,95(t), 3,33(s), 4,55(d), 5,35(dd), 5,36(s) |
| 21 | $CH(OCH_3)_2$ | n-$C_3H_7$ | (E)-$CH_2-CH=CHCl$ | 0,99(t), 3,33(s), 4,53(d), 5,38(s) |
| 22 | $CH(OCH_3)_2$ | n-$C_3H_7$ | (E)-$CH_2-CH=CH-CH_3$ | 1,0(t), 1,78(d), 3,34(s), 5,39(s) |
| 23 | $CH(OC_2H_5)_2$ | $C_2H_5$ | $C_2H_5$ | 2,97(q), 4,15(q), 5,5(s) |
| 24 | $CH(OC_2H_5)_2$ | $C_2H_5$ | $CH_2-CH=CH_2$ | |
| 25 | $CH(OC_2H_5)_2$ | $C_2H_5$ | (E)-$CH_2-CH=CHCl$ | |
| 26 | $CH(OC_2H_5)_2$ | $C_2H_5$ | (E)-$CH_2-CH=CH-CH_3$ | |
| 27 | $CH(OC_2H_5)_2$ | n-$C_3H_7$ | $C_2H_5$ | 0,99(t), 1,23(t), 1,32(t), 4,12(q), 5,5(s) |
| 28 | $CH(OC_2H_5)_2$ | n-$C_3H_7$ | $CH_2-CH=CH_2$ | |
| 29 | $CH(OC_2H_5)_2$ | n-$C_3H_7$ | (E)-$CH_2-CH=CHCl$ | |
| 30 | $CH(OC_2H_5)_2$ | n-$C_3H_7$ | (E)-$CH_2-CH=CHCH_3$ | |
| 31 | $CH(OC_4H_9)_2$ | $C_2H_5$ | $C_2H_5$ | |
| 32 | $CH(OC_4H_9)_2$ | $C_2H_5$ | $CH_2-CH=CH_2$ | |
| 33 | $CH(OC_4H_9)_2$ | $C_2H_5$ | (E)-$CH_2-CH=CHCl$ | |
| 34 | $CH(OC_4H_9)_2$ | $C_2H_5$ | (E)-$CH_2-CH=CHCH_3$ | |
| 35 | $CH(OC_4H_9)_2$ | n-$C_3H_7$ | $C_2H_5$ | |

Tabelle (Fortsetzung)

| Wirkstoff Nr. | $R^3$ | $R^2$ | $R^1$ | Smp. [°C] bzw. charakterist. $^1$H-NMR-Daten [δ ppm] |
|---|---|---|---|---|
| 36 | $CH(OC_4H_9)_2$ | n-$C_3H_7$ | $CH_2$-CH=$CH_2$ | |
| 37 | $CH(OC_4H_9)_2$ | n-$C_3H_7$ | (E)-$CH_2$-CH=CHCl | |
| 38 | $CH(OC_4H_9)_2$ | n-$C_3H_7$ | (E)-$CH_2$-CH=$CHCH_3$ | |
| 39 | $CH(OCH_3)_2$ | n-$C_3H_7$ | n-$C_3H_7$ | 0,99(t), 3,34(s), 4,04(t), 5,37(s) |
| 40 | $CH(OCH_3)_2$ | n-$C_3H_7$ | $CH_2$-C≡CH | 0.97(t), 3,34(s), 4,66(d), 5,34(s) |
| 41 | CHO | n-$C_3H_7$ | 5-Chlor-2-thenyl | |
| 42 | CHO | $C_2H_5$ | 5-Chlor-2-thenyl | |
| 43 | $CH(OCH_3)_2$ | n-$C_3H_7$ | 5-Chlor-2-thenyl | |
| 44 | $CH(OCH_3)_2$ | $C_2H_5$ | 5-Chlor-2-thenyl | |
| 45 | $CH(OCH_2CH_2OCH_3)_2$ | n-$C_3H_7$ | $C_2H_5$ | |
| 46 | $CH(OCH_2CH_2OCH_3)_2$ | $C_2H_5$ | (E)-$CH_2$-CH=CH-$CH_3$ | |
| 47 | $CH(OCH_2CH_2SCH_2CH_3)_2$ | n-$C_3H_7$ | $C_2H_5$ | |
| 48 | $CH(OCH_2CH_2SCH_2CH_3)_2$ | $C_2H_5$ | $CH_2$-CH=$CH_2$ | |
| 49 | $CH(OCH_2CH_2Cl)_2$ | n-$C_3H_7$ | $C_2H_5$ | |
| 50 | $CH(OCH_2CH_2Cl)_2$ | n-$C_3H_7$ | $CH_2$-CH=$CH_2$ | |
| 51 | $CH(OCH_2CH_2Cl)_2$ | n-$C_3H_7$ | (E)-$CH_2$-CH=CH-Cl | |
| 52 | $CH(OCH_2CH_2Cl)_2$ | $C_2H_5$ | $C_2H_5$ | |
| 53 | $CH(OCH_2CH_2Cl)_2$ | $C_2H_5$ | (E)-$CH_2$-CH=CH-$CH_3$ | |
| 54 | $CH(OCH_2CCl_3)_2$ | $C_2H_5$ | (E)-$CH_2$-CH=CH-$CH_3$ | |
| 55 | $CH(OCH_2CH_2CH_2Br)_2$ | n-$C_3H_7$ | $C_2H_5$ | |
| 56 | $CH(OCH_2CH_2CH_2Br)_2$ | $C_2H_5$ | (E)-$CH_2$-CH=CH-Cl | |
| 57 | $CH(OCH_2CF_2CF_2CF_3)_2$ | n-$C_3H_7$ | (E)-$CH_2$-CH=CH-Cl | |
| 58 | $CH(OCH_2CF_2CF_2CF_3)_2$ | $C_2H_5$ | (E)-$CH_2$-CH=CH-$CH_3$ | |
| 59 | $CH(OCH(CH_3)CH_2OCH_3)_2$ | $C_2H_5$ | $C_2H_5$ | |
| 60 | $CH(SCH_3)_2$ | n-$C_3H_7$ | $C_2H_5$ | 0,99(t), 1,32(t), 2,1(s), 4,79(s) |
| 61 | $CH(SCH_3)_2$ | n-$C_3H_7$ | (E)-$CH_2$-CH=CH-$CH_3$ | 0,99(t), 2,1(t), 4,47(d), 4,79(s) |

Tabelle (Fortsetzung)

| Wirkstoff Nr. | $R^3$ | $R^2$ | $R^1$ | Smp. [°C] bzw. charakterist. $^1$H-NMR-Daten [δ ppm] |
|---|---|---|---|---|
| 62 | $CH(SCH_3)_2$ | n-$C_3H_7$ | (E)-$CH_2$-CH=CH-Cl | 0,98(t), 2,11(s), 4,56(d), 4,78(s) |
| 63 | $CH(SCH_3)_2$ | $C_2H_5$ | $C_2H_5$ | 69-71 |
| 64 | $CH(SCH_3)_2$ | $C_2H_5$ | n-$C_3H_7$ | |
| 65 | $CH(SCH_2CH_3)_2$ | $C_2H_5$ | $C_2H_5$ | 1.17(t,3H), 1.23(t,6H), 1.34(t,3H), 4.1 (q,2H), 4.92 (s,1H) |
| 66 | $CH(SCH_2CH_3)_2$ | n-$C_3H_7$ | $C_2H_5$ | 1.0 (t,3H), 1.26(t,6H), 1.36(t,3H), 1.61 ("6",2H), 4.16(q,2H) |
| 67 | $CH(SCH_2CH_2CH_3)_2$ | n-$C_3H_7$ | $C_2H_5$ | |
| 68 | $CH(SCH_2CH_2CH_3)_2$ | $C_2H_5$ | (E)-$CH_2$-CH=CH-$CH_3$ | 0,95(t), 1,14(t), 4,47(d), 4,86(s) |
| 69 | $CH(SCH_2CH_2CH_2CH_3)_2$ | n-$C_3H_7$ | $C_2H_5$ | |
| 70 | $CH(SCH_2CH_2CH_2CH_3)_2$ | n-$C_3H_7$ | $CH_2$-C≡CH | |
| 71 | $CH(SCH_2CH_2SCH_2CH_3)_2$ | n-$C_3H_7$ | (E)-$CH_2$-CH=CH-$CH_3$ | |
| 72 | $CH(SCH_2CH_2SCH_2CH_3)_2$ | $C_2H_5$ | (E)-$CH_2$-CH=CH-Cl | |
| 73 | $CH(SCH_2CH_2OCH_2CH_3)_2$ | $C_2H_5$ | (E)-$CH_2$-CH=CH-$CH_3$ | |
| 74 | $CH(SCH_2CH_2OCH_2CH_3)_2$ | n-$C_3H_7$ | $CH_2$-CH=$CH_2$ | |
| 75 | $CH(SCH_2CH_2CH_2Cl)_2$ | n-$C_3H_7$ | $C_2H_5$ | |
| 76 | $CH(SCH_2CH_2CH_2Cl)_2$ | $C_2H_5$ | $CH_2$-CH=$CH_2$ | |
| 77 | $CH(SCH_2CH_2CN)_2$ | n-$C_3H_7$ | (E)-$CH_2$-CH=CH-Cl | |
| 78 | $CH(SCH_2CH_2CN)_2$ | $C_2H_5$ | (E)-$CH_2$-CH=CH-$CH_3$ | |
| 79 | $CH[SCH_2CH_2N(CH_3)_2]_2$ | n-$C_3H_7$ | $C_2H_5$ | |
| 80 | $CH[SCH_2CH_2N(CH_3)_2]_2$ | $C_2H_5$ | $C_2H_5$ | |
| 81 | 1,3-Dioxolan-2-yl | n-$C_3H_7$ | $C_2H_5$ | 94-95 |
| 82 | 1,3-Dioxolan-2-yl | $C_2H_5$ | (E)-$CH_2$-CH=CH-Cl | |
| 83 | 4,4-Dimethyl-1,3-dioxolan-2-yl | n-$C_3H_7$ | n-$C_3H_7$ | |
| 84 | 4,4-Dimethyl-1,3-dioxolan-2-yl | $C_2H_5$ | $CH_2$-CH=$CH_2$ | |

Tabelle (Fortsetzung)

| Wirkstoff Nr. | $R^3$ | $R^2$ | $R^1$ | Smp.[.°C] bzw. charakterist. $^1$H-NMR-Daten [δ ppm] |
|---|---|---|---|---|
| 85 | 4-Chlormethyl-1,3-dioxolan-2-yl | $n\text{-}C_3H_7$ | $C_2H_5$ | |
| 86 | 4-Chlormethyl-1,3-dioxolan-2-yl | $n\text{-}C_3H_7$ | (E)-$CH_2$-CH=CH-$CH_3$ | |
| 87 | 4-Chlormethyl-1,3-dioxolan-2-yl | $C_2H_5$ | $C_2H_5$ | |
| 88 | 4-Methylthioethyl-1,3-dioxolan-2-yl | $n\text{-}C_3H_7$ | $C_2H_5$ | |
| 89 | 4-Hydroxymethyl-1,3-dioxolan-2-yl | $n\text{-}C_3H_7$ | $C_2H_5$ | |
| 90 | 4-Hydroxymethyl-1,3-dioxolan-2-yl | $C_2H_5$ | $C_2H_5$ | |
| 91 | 1,3-Dithiolan-2-yl | $n\text{-}C_3H_7$ | $C_2H_5$ | 0.99(t,3H), 1.34(t,3H), 1.61(m,2H), 4.12(g,2H), 5.66(s,1H) |
| 92 | 1,3-Dithiolan-2-yl | $n\text{-}C_3H_7$ | $CH_2$-CH=$CH_2$ | 1.01(t,3H), 1.62(m,2H), 4.58(d,2H), 5.67(s,1H) |
| 93 | 1,3-Dithiolan-2-yl | $C_2H_5$ | (E)-$CH_2$-CH=CH-$CH_3$ | 97-98 |
| 94 | 1,3-Dithiolan-2-yl | $C_2H_5$ | (E)-$CH_2$-CH=CH-Cl | 72-75 |
| 95 | 4-Methyl-1,3-dithiolan-2-yl | $C_2H_5$ | $C_2H_5$ | |
| 96 | 1,3-Oxothiolan-2-yl | $n\text{-}C_3H_7$ | $C_2H_5$ | |
| 97 | 1,3-Dithiolan-2-yl | $n\text{-}C_3H_7$ | $CH_2$-C≡CH | |
| 98 | 1,3-Dithiolan-2-yl | $n\text{-}C_3H_7$ | (E)-$CH_2$-CH=CH-$CH_3$ | |
| 99 | 5,5-Dimethyl-1,3-dioxan-2-yl | $n\text{-}C_3H_7$ | $C_2H_5$ | 94-95 |
| 100 | 5,5-Dimethyl-1,3-dioxan-2-yl | $n\text{-}C_3H_7$ | (E)-$CH_2$-CH=CH-Cl | 99-100 |
| 101 | 1,3-Dioxan-2-yl | $n\text{-}C_3H_7$ | $CH_2$-CH=$CH_2$ | |
| 102 | 1,3-Dioxan-2-yl | $C_2H_5$ | (E)-$CH_2$-CH=CH-Cl | |
| 103 | 1,3-Dioxan-2-yl | $C_2H_5$ | (E)-$CH_2$-CH=CH-$CH_3$ | |
| 104 | 4,4,6-Trimethyl-1,3-dioxan-2-yl | $n\text{-}C_3H_7$ | $C_2H_5$ | |
| 105 | 4,4,6-Trimethyl-1,3-dioxan-2-yl | $C_2H_5$ | $C_2H_5$ | |
| 106 | 5-Methoxymethyl-5-methyl-1,3-dioxan-2-yl | $n\text{-}C_3H_7$ | $C_2H_5$ | |

Tabelle (Fortsetzung)

| Wirkstoff Nr. | $R^3$ | $R^2$ | $R^1$ | Smp. [°C] bzw. charakterist. $^1$H-NMR-Daten [δ ppm] |
|---|---|---|---|---|
| 107 | 1,3-Dithian-2-yl | $n-C_3H_7$ | $C_2H_5$ | 1,0(t), 1,33(t), 4,11(q), 5,17(s) |
| 108 | 1,3-Dithian-2-yl | $n-C_3H_7$ | (E)-$CH_2$-CH=CH-Cl | 1,0(t), 4,52(d), 5,18(s) |
| 109 | 1,3-Dithian-2-yl | $C_2H_5$ | $CH_2$-CH=$CH_2$ | 78-81 |
| 110 | 1,3-Dithian-2-yl | $C_2H_5$ | (E)-$CH_2$-CH=CH-$CH_3$ | 121-122 |
| 111 | $CH(OCH_3)_2$ Na$^+$-Salz | $n-C_3H_7$ | $C_2H_5$ | |
| 112 | 1,3-Dithiolan-2-yl | $C_2H_5$ | $CH_2$-CH=$CH_2$ | 65-68 |
| 113 | 1,3-Dithiolan-2-yl | $C_2H_5$ | $C_2H_5$ | 84-87 |
| 114 | 1,3-Dithian-2-yl | $C_2H_5$ | $C_2H_5$ | 143-45 |
| 115 | 1,3-Dithian-2-yl | $C_2H_5$ | (E)-$CH_2$-CH=CH-Cl | 111-14 |
| 116 | $CH(OCH_3)_2$ | $C_2H_5$ | $n-C_3H_7$ | |
| 117 | $CH(OCH_3)_2$ | $C_2H_5$ | $CH_2C{\equiv}CH$ | 1.18(t,3H), 3.32(s,6H), 4.68(d,2H), 5,39(s,1H) |
| 118 | $CH(OCH_3)_2$ | $n-C_3H_7$ | $CH_2C{\equiv}CCH_3$ | 0.97(t,3H), 3.35(s,6H), 5.38(s,1H), 7.26(d,2H), 7.44(d,2H) |
| 119 | $CH(SCH_3)_2$ | $C_2H_5$ | $CH_2CH=CH_2$ | 1.2(t,3H), 2.11(s,6H), 4.59(d,2H), 4.8(s,1H) |
| 120 | $CH(SCH_3)_2$ | $C_2H_5$ | $CH_2CH=CHCH_3$ | 1.17(t,3H), 1.79(d,3H), 2.12(s,6H), 4.79(s,1H) |
| 121 | $CH(SCH_3)_2$ | $C_2H_5$ | $CH_2CH=CHCl$ | 1.18(t,3H), 2.12(s,6H), 4.56(d,2H), 4.79(s,1H) |
| 122 | $CH(SCH_3)_2$ | $n-C_3H_7$ | $CH_2CH=CH_2$ | 0.98(t,3H), 2.12(s,6H), 4.56(d,2H), 4.77(s,1H), 5.87-6.08(m,1H) |
| 123 | 1,3-Dioxolan-2-yl | $n-C_3H_7$ | $CH_2CH=CHCH_3$ | 84-85 |
| 124 | 1,3-Dioxolan-2-yl | $n-C_3H_7$ | $CH_2CH=CHCl$ | 78-80 |

Tabelle (Fortsetzung)

| Wirkstoff Nr. | $R^3$ | $R^2$ | $R^1$ | Smp. [°C] bzw. charakterist. $^1$H-NMR-Daten [δ ppm] |
|---|---|---|---|---|
| 125 | 1,3-Dioxan-2-yl | n-$C_3H_7$ | $CH_2CH{=}CHCH_3$ | 108-110 |
| 126 | 5,5-Dimethyl-1,3-dioxan-2-yl | $C_2H_5$ | $C_2H_5$ | 115-116 |

Die Applikation der Mittel kann im Vorauflaufverfahren oder im Nachauflaufverfahren erfolgen. Sind die Wirkstoffe für gewisse Kulturpflanzen weniger verträglich, so können auch Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen

(post-directed, lay-by).

Die Aufwandmengen an Wirkstoff betragen je nach Jahreszeit, Zielpflanzen und Wachstumsstadium 0,01 bis 3 kg/ha, vorzugsweise 0,05 bis 1,0 kg/ha.

Die Wirkung der Wirkstoffe der Formel I auf das Pflanzenwachstum läßt sich durch Gewächshausversuche zeigen:

Als Kulturgefäße dienen Plastikblumentöpfe mit 300 $cm^3$ Inhalt und lehmigem Sand mit etwa 3,0 % Humus als Substrat. Die Samen der Testpflanzen werden nach Arten getrennt flach eingesät. Bei Vorauflaufbehandlung werden die aufbereiteten Wirkstoffe unmittelbar danach auf die Erdoberfläche aufgebracht. Sie werden hierbei in Wasser als Verteilungsmittel suspendiert oder emulgiert und mittels fein verteilender Düsen gespritzt. Die Aufwandmengen betragen 0,5 kg/ha.

Nach dem Aufbringen der Mittel werden die Gefäße leicht beregnet, um Keimung und Wachstum in Gang zu bringen. Danach deckt man die Gefäße mit durchsichtigen Plastikhauben ab, bis die Pflanzen angewachsen sind. Diese Abdeckung bewirkt ein gleichmäßiges Keimen der Testpflanzen, sofern dies nicht durch die Wirkstoffe beeinträchtigt wird.

Zum Zwecke der Nachauflaufbehandlung zieht man die Testpflanzen je nach Wuchsform erst bis zu einer Wuchshöhe von 3 bis 15 cm an und behandelt sie danach. Zur Nachauflaufbehandlung werden entweder direkt gesäte und in den gleichen Gefäßen aufgewachsene Pflanzen ausgewählt, oder sie werden erst als Keimpflanzen getrennt angezogen und einige Tage vor der Behandlung in die Versuchsgefäße verpflanzt. Die Aufwandmengen für die Nachauflaufbehandlung betragen 0,06, 0,125 und 0,25 kg Wirkstoff/ha. Eine Abdeckung unterbleibt bei der Nachauflaufbehandlung.

Die Versuchsgefäße werden im Gewächshaus aufgestellt, wobei für wärmeliebende Arten wärmere Bereiche (20 bis 35 °C) und für solche gemäßigter Klimate 10 bis 25 °C bevorzugt werden. Die Versuchsperiode erstreckt sich über 2 bis 4 Wochen. Während dieser Zeit werden die Pflanzen gepflegt und ihre Reaktion auf die einzelnen Behandlungen wird ausgewertet. Bewertet wird nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen gehören den nachstehenden Arten an:

| Abkürzung | Lateinischer Name | Deutscher Name | Englischer Name |
|---|---|---|---|
| AVEFA | Avena fatua | Flughafer | wild oats |
| ALOMY | Alopecurus myosuroides | Ackerfuchsschwanz | blackgrass |
| BROIN | Bromus inermis | unbegrannte Trespe | smooth broome |
| DIGSA | Digitaria sanguinalis | Blutfingerhirse | large crabgrass |
| LOLMU | Lolium multiflorum | Ital. Raygras | annual raygrass |
| MEDSA | Medicago sativa | Luzerne | alfalfa |
| SETIT | Setaria italica | Kolbenhirse | foxtail millet |
| SETVI | Setaria viridis | Grüne Borstenhirse | green foxtail |
| SINAL | Sinapis alba | Weißer Senf | white mustard |
| TRZAS | Triticum aestivum | Weizen | wheat |
| TRZAW | Triticum aestivum | Weizen | wheat |
| ZEAMX | Zea mays | Mais | Indian corn |

Zur Bekämpfung grasartiger Vegetation eignen sich die Bsp. 8, 111 und 19 bei Nachauflaufanwendung von 0,25 kg a.S./ha. Breitblättrige Kulturen, wie am Beispiel von Luzerne gezeigt, werden dabei nicht geschädigt. Die neuen Wirkstoffe haben selektive herbizide Wirkung (Tabelle 1).

Der Wirkstoff 8 kann im Nachauflaufverfahren zur Bekämpfung unerwünschter Grasarten in Weizen eingesetzt werden. Die Nutzpflanzen erleiden eine allenfalls geringe Schädigung (Tab. 2).

Die Wirkstoffe 8 und 19 zeigen bei Vorauflaufanwendung von 0,5 kg a.S./ha starke herbizide Wirkung gegen grasartige Beispielpflanzen, Senf als breitblättrige Spezies bleibt unbeeinflußt (Tab. 3).

Die Verbindungen Nr. 3 (Tab. 4) und Nr. 4 (Tab. 5) wirken ebenfalls gegen unerwünschte Gräser ohne oder mit sehr geringer Beeintrichtigung des Wachstums der Weizenpflanzen. Die Beispielverbindungen Nr. 5 und Nr. 17 (Tab. 6) bekämpfen ebenso unerwünschte grasartige Pflanzen, ohne Luzerne als Beispielpflanze für breitblättrige Kulturen zu schädigen.

Tabelle 1
Herbizide Wirkung gegen unerwünschte grasartige Pflanzen sowie Verträglichkeit für eine Beispielkultur bei Nachauflaufanwendung im Gewächshaus (0,25 kg/ha a.S.)

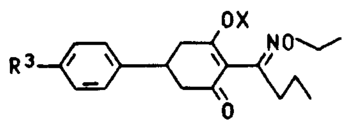

| Bsp. Nr. | $R^3$ | X | MEDSA | AVEFA | BROIN | LOLMU | ZEAMX* |
|---|---|---|---|---|---|---|---|
| 8 | CHO | H | 0 | 100 | 95 | 100 | 100 |
| 111 | $CH(OCH_3)_2$ | Na | 0 | 100 | 98 | 98 | 98 |
| 19 | $CH(OCH_3)_2$ | H | 0 | 100 | 98 | 100 | 100 |

* als Ausfallmais

Tabelle 2

Herbizide Wirkung gegen unerwünschte grasartige Pflanzen sowie Verträglichkeit für eine Beispielkultur bei Nachauflaufanwendung im Gewächshaus

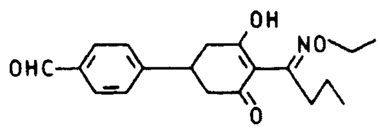

| Bsp.Nr. | kg a.S./ha | Testpflanzen und Schädigung % | | |
|---|---|---|---|---|
| | | TRZAS* | ALOMY | AVEFA |
| 8 | 0,06 | 5 | 98 | 95 |

* Sorte Schirokko

Tabelle 3

Herbizide Wirkung gegen unerwünschte grasartige Pflanzen und Verträglichkeit für eine breitblättrige Beispielspflanze bei Vorauflaufanwendung von 0,5 kg a.S./ha im Gewächshaus

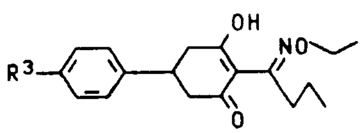

| Bsp.Nr. | $R^3$ | Testpflanzen und Schädigung % | |
|---|---|---|---|
| | | SINAL | LOLMU |
| 8 | CHO | 0 | 100 |
| 19 | $CH(OCH_3)_2$ | 0 | 100 |

Beispiele zur Bekämpfung unerwünschter grasartiger Pflanzen und Verträglichkeit für eine Beispielkultur bei Nachauflaufapplikation von 0,125 kg/ha a.S. im Gewächshaus

Tabelle 4

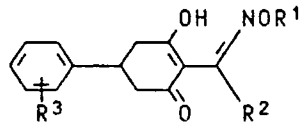

| Bsp.-Nr. | $R_1$ | $R_2$ | $R_3$ | TRZAS | LOLMU | DIGSA |
|---|---|---|---|---|---|---|
| 3 | $CH_2CH{=}CH_2$ | $C_2H_5$ | 4-CHO | 10 | 100 | 95 |

Tabelle 5

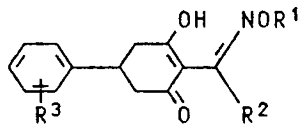

| Bsp.-Nr. | $R_1$ | $R_2$ | $R_3$ | TRZAS | DIGSA | SETIT |
|---|---|---|---|---|---|---|
| 4 | (E)-$CH_2CH{=}CHCl$ | $C_2H_5$ | 4-CHO | 0 | 95 | 100 |

Tabelle 6

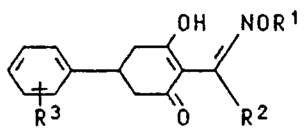

| Bsp.-Nr. | $R_1$ | $R_2$ | $R_3$ | MEDSA | LOLMU | DIGSA | SETVI |
|---|---|---|---|---|---|---|---|
| 5 | (E)-$CH_2CH{=}CHCH_3$ | $C_2H_5$ | 4-CHO | 0 | 100 | 100 | 100 |
| 17 | (E)-$CH_2CH{=}CHCl$ | $C_2H_5$ | 4-$CH(OCH_3)_2$ | 0 | 100 | 98 | 100 |

In Anbetracht des erfaßbaren Wirkungsspektrums zur Unkrautbekämpfung, der Verträglichkeit für Kulturpflanzen oder der erwünschten Beeinflussung des Wachstums derselben sowie angesichts der Vielfalt der Applikationsmethoden können die erfindungsgemäßen Verbindungen in einer großen Zahl von Kulturpflanzen eingesetzt werden. In Betracht kommen beispielsweise folgende Kulturen:

| Botanischer Name | Deutscher Name |
| --- | --- |
| Allium cepa | Küchenzwiebel |
| Ananas comosus | Ananas |
| Arachis hypogaea | Erdnuß |
| Asparagus officinalis | Spargel |
| Avena sativa | Hafer |
| Beta vulgaris spp. altissima | Zuckerrübe |
| Beta vulgaris spp. rapa | Futterrübe |
| Beta vulgaris spp. esculenta | Rote Rübe |
| Brassica napus var. napus | Raps |
| Brassica napus var. napobrassica | Kohlrübe |
| Brassica napus var. rapa | Weiße Rübe |
| Brassica rapa var. silvestris | Rüben |
| Camellia sinensis | Teestrauch |
| Carthamus tinctorius | Saflor - Färberdistel |
| Carya illinoinensis | Pekannußbaum |
| Citrus limon | Zitrone |
| Citrus maxima | Pampelmuse |
| Citrus reticulata | Mandarine |
| Citrus sinensis | Apfelsine, Orange |
| Coffea arabica (C.canephora, Coffea liberica) | Kaffee |
| Cucumis melo | Melone |
| Cucumis sativus | Gurke |
| Cynodon dactylon | Bermudagras |
| Daucus carota | Möhre |
| Elaeis guineensis | Ölpalme |
| Fragaria vesca | Erdbeere |
| Glycine max | Sojabohne |

| Botanischer Name | Deutscher Name |
|---|---|
| Gossypium hirsutum (G.arboreum, G., G. vitifolium) | Baumwolle |
| Helianthus annuus | Sonnenblume |
| Helianthus tuberosus | Topinambur |
| Hevea brasiliensis | Parakautschukbaum |
| Hordeum vulgare | Gerste |
| Humulus lupulus | Hopfen |
| Ipomoea batatas | Süßkartoffeln |
| Juglans regia | Walnußbaum |
| Lactuca sativa | Kopfsalat |
| Lens culinaris | Linse |
| Linum usitatissimum | Faserlein |
| Lycopersicon lycopersicum | Tomate |
| Malus spp. | Apfel |
| Manihot esculenta | Maniok |
| Medicago sativa | Luzerne |
| Mentha piperita | Pfefferminze |
| Musa spp. | Obst- und Mehlbanane |
| Nicotiana tabacum (N. rustica) | Tabak |
| Olea europaea | Ölbaum |
| Oryza sativa | Reis |
| Panicum miliaceum | Rispenhirse |
| Phaseolus lunatus | Mondbohne |
| Phaseolus mungo | Erdbohne |
| Phaseolus vulgaris | Buschbohnen |
| Pennisetum glaucum | Perl- oder Rohrkolbenhirse |
| Petroselinum crispum spp. tuberosum | Wurzelpetersilie |
| Picea abies | Rotfichte |
| Abies alba | Weißtanne |
| Pinus spp. | Kiefer |
| Pisum sativum | Gartenerbse |
| Prunus avium | Süßkirsche |
| Prunus domestica | Pflaume |
| Prunus dulcis | Mandelbaum |
| Prunus persica | Pfirsich |
| Pyrus communis | Birne |
| Ribes sylvestre | Rote Johannisbeere |
| Ribes uva-crispa | Stachelbeere |
| Ricinus communis | Rizinus |
| Saccharum officinarum | Zuckerrohr |
| Secale cereale | Roggen |
| Sesamum indicum | Sesam |

| Botanischer Name | Deutscher Name |
|---|---|
| Solanum tuberosum | Kartoffel |
| Sorghum bicolor (S. vulgare) | Mohrenhirse |
| Sorghum dochna | Zuckerhirse |
| Spinacia oleracea | Spinat |
| Theobroma cacao | Kakaobaum |
| Trifolium pratense | Rotklee |
| Triticum aestivum | Weizen |
| Vaccinium corymbosum | Kulturheidelbeere |
| Vaccinium vitis-idaea | Preißelbeere |
| Vicia faba | Pferdebohnen |
| Vigna sinensis (V. unguiculata) | Kuhbohne |
| Vitis vinifera | Weinrebe |
| Zea mays | Mais |

Zur Verbreiterung des Wirkungsspektrums und zur Erzielung synergistischer Effekte können die Wirkstoffe der Formel I sowohl unter sich als auch mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Cyclohexenone, Triazinone, Uracile, Benzofuranderivate, Chinolincarbonsäuren, (Hetero)-aryloxy-phenoxypropionsäuren sowie deren Salze, Ester und Amide in Betracht.

Außerdem kann es von Nutzen sein, die Wirkstoffe der Formel I allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

## Patentansprüche

**1.** Cyclohexenonverbindung der Formel I

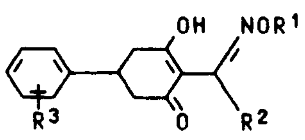

(I),

in der die Substituenten folgende Bedeutung haben

- $R^1$ Alkyl mit 1 bis 4 C-Atomen, Alkenyl oder Alkinyl mit 3 oder 4 C-Atomen, Haloalkenyl mit 3 oder 4 C-Atomen und 1 bis 3 Halogensubstituenten, ggf. bis zu dreifach halo- und/oder $C_1$-$C_4$-alkylsubstituiertes Thenyl,
- $R^2$ Alkyl mit 1 bis 4 C-Atomen
- $R^3$ Formyl, ein Rest der allgemeinen Formel $R^4XCHXR^5$, wobei X = O oder S und $R^4$, $R^5$ gleiches oder verschiedenes Alkyl oder gemeinsam Alkylen mit jeweils 1 bis 4 C-Atomen bedeutet und der zusätzlich durch $C_1$-$C_4$-Alkyl, $C_1$-$C_4$-Alkoxy, $C_1$-$C_4$-Alkylthio, Hydroxy, Halogen, Cyano, $C_1$-$C_4$-N,N-Dialkylamino substituiert sein kann,

sowie ihre biologisch wirksamen Salze mit Alkalimetall-, Erdalkalimetall- und Ammoniumionen und ihre Ester mit $C_1$-$C_{10}$-Carbonsäuren oder anorganischen Säuren.

**2.** Verfahren zur Herstellung von Cyclohexenonverbindungen der Formel I gemäß Anspruch 1, dadurch gekennzeichnet, daß man eine entsprechende Cyclohexenonverbindung der Formel II

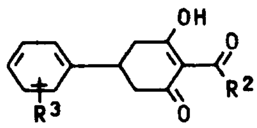

(II)

in der $R^3$ nicht die Bedeutung Formyl hat, mit einer entsprechenden Hydroxylaminverbindung $R^1ONH_2$ bzw. der entsprechenden Hydroxylammoniumverbindung in Gegenwart einer Base in an sich bekannter Weise umsetzt und, wenn $R^3$ in Formel I die Bedeutung CHO haben soll, das erhaltene Zwischenprodukt gegebenenfalls mit einer Säure behandelt.

**3.** Verfahren zur Herstellung einer Cyclohexenonverbindung der Formel I gemäß Anspruch 1, in der $R^3$ die Bedeutung Formyl hat, dadurch gekennzeichnet, daß man einen entsprechenden Cyclohexenonester der Formel III,

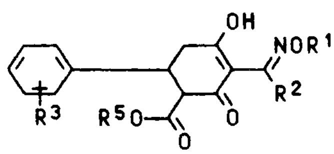

(III)

in der $R^5$ Alkyl mit 1 bis 4 C-Atomen bedeutet, zunächst alkalisch verseift und ohne Isolierung der Carbonsäure durch anschließendes Ansäuern gleichzeitig decarboxyliert und die Acetalfunktion $R^3$ in die Formylfunktion überführt.

**4.** Herbizid, enthaltend eine Cyclohexenonverbindung der Formel I gemäß Anspruch 1.

**5.** Herbizid, enthaltend übliche Hilfsmittel und eine Cyclohexenonverbindung der Formel I gemäß Anspruch 1.

**6.** Verfahren zur Bekämpfung unerwünschten Pflanzenwuchses, dadurch gekennzeichnet, daß man die unerwünschten Pflanzen und/oder die von unerwünschtem Pflanzenwachstum freizuhaltende Fläche mit einer herbizid wirksamen Menge einer Cyclohexenonverbindung der Formel I gemäß Anspruch 1 behandelt.

## Claims

**1.** A cyclohexenone compound of the formula I

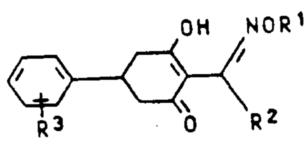

(I)

where $R^1$ is alkyl of 1 to 4 carbon atoms, alkenyl or alkynyl of 3 or 4 carbon atoms, haloalkenyl of 3 or 4 carbon atoms and 1 to 3 halogen substituents, or is thenyl which is unsubstituted or mono-, di- or trisubstituted by halo and/or $C_1$-$C_4$-alkyl, $R^2$ is alkyl of 1 to 4 carbon atoms, and $R^3$ is formyl, a radical of the formula $R^4XCHXR^5$, where X is oxygen or sulfur, and $R^4$ and $R^5$ are identical or different alkyl, or together are alkylene of 1 to 4 carbon atoms in each case and which additionally can be substituted by $C_1$-$C_4$-alkyl, $C_1$-$C_4$-alkoxy, $C_1$-$C_4$-alkylthio, hydroxy, halogen, cyano, or $C_1$-$C_4$-N,N-dialkylamino, or a biologically active salt thereof with an alkali metal, alkaline earth metal or ammonium ion or an ester thereof with a $C_1$-$C_{10}$-carboxylic acid or an inorganic acid.

**2.** A process for the preparation of a cyclohexenone compound of the formula I as claimed in claim 1, wherein a corresponding cyclohexenone compound of the formula II

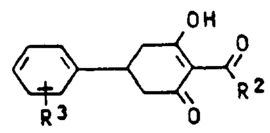

(II),

where $R^3$ is not formyl, is reacted in conventional manner with a corresponding hydroxylamine compound $R^1ONH_2$ or the corresponding hydroxylammonium compound in the presence of a base, and, if $R^3$ in formula I is to be CHO, the intermediate obtained is if desired treated with an acid.

**3.** A process for the preparation of a cyclohexenone compound of the formula I as claimed in claim 1, where $R^3$ is formyl, wherein a corresponding cyclohexenone ester of the formula III

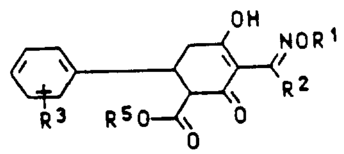

(III)

where $R^5$ is alkyl of 1 to 4 carbon atoms, is first subjected to alkaline saponification and, without isolation of the carboxylic acid, carboxylated by subsequent acidification while the acetal function $R^3$ is simultaneously converted into the formyl function.

**4.** A herbicide containing a cycohexenone compound of the formula I as claimed in claim 1.

**5.** A herbicide containing conventional auxiliaries and a cyclohexenone compound of the formula I as claimed in claim 1.

**6.** A process for controlling undesired plant growth, which comprises treating the undesired plants and/or the area to be kept free from undesired plant growth with a herbicidally effective amount of a cyclohexenone compound of the formula I as claimed in claim 1.

**Revendications**

**1.** Dérivé de cyclohexénone de formule I

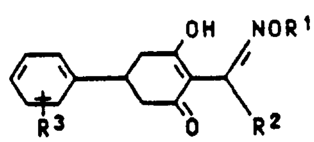

(I),

dans laquelle les symboles ont les significations suivantes :

$R^1$ représente un groupe alkyle en C 1-C 4, un groupe alcényle ou alcynyle en C 3-C 4, un groupe halogénoalcényle contenant 3 ou 4 atomes de carbone et 1 à 3 atomes d'halogènes substituants, un groupe thényle portant éventuellement jusqu'à 3 substituants halogéno et/ou alkyle en C 1-C 4,

$R^2$ représente un groupe alkyle en C 1-C 4,

$R^3$ représente un groupe formyle, un groupe de formule générale $R^4XCHXR^5$ dans laquelle X = O ou S et $R^4$, $R^5$ représentent des groupes alkyle identiques ou différents en C 1-C 4 ou forment ensemble un groupe alkylène en C 1-C 4 et qui peut en outre être substitué par des groupes alkyle en C 1-C 4, alcoxy en C 1-C 4, alkylthio en C 1-C 4, hydroxy, des halogènes, des groupes cyano, N,N-di-(alkyle en C 1-C 4)-amino,

et leurs sels, possédant une activité biologique, d'ions de métaux alcalins, de métaux alcalino-terreux et

d'ammonium et leurs esters d'acides carboxyliques en C 1-C 10 ou d'acides minéraux.

2. Procédé de préparation des dérivés de cyclohexénone de formule I de la revendicationn 1, caractérisé en ce que l'on fait réagir de manière connue en soi, en présence d'une base, un dérivé correspondant de cyclohexénone de formule II

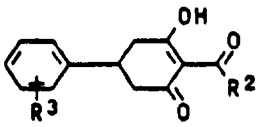

(II)

dans laquelle $R^3$ a une signification autre que le groupe formyle, avec un dérivé correspondant d'hydroxylamine $R^1OHN_2$ ou le dérivé d'hydroxylammonium correspondant et, lorsque $R^3$ de la formule I doit représenter CHO, on traite éventuellement le produit intermédiaire obtenu par un acide.

3. Procédé de préparation d'un dérivé de cyclohexénone de formule I de la revendication 1 dans laquelle $R^3$ représente un groupe formyle, caractérisé en ce que l'on soumet d'abord un ester de cyclohexénone correspondant de formule III

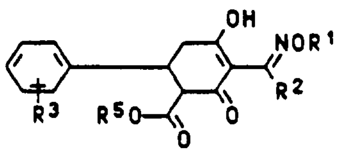

(III)

dans laquelle $R^5$ représente un groupe alkyle en C 1-C 4, à une saponification alcaline, et sans isoler l'acide carboxylique, on provoque simultanément, par une acidification subséquente, la décarboxylation et la conversion de la fonction acétal $R^3$ en la fonction formyle.

4. Produit herbicide contenant un dérivé de cyclohexénone de formule I de la revendication 1.

5. Produit herbicide contenant des produits auxiliaires usuels et un dérivé de cyclohexénone de formule I de la revendication 1.

6. Procédé pour combattre les croissances de végétaux indésirables, caractérisé en ce que l'on traite les végétaux indésirables et/ou la surface à débarasser des croissances de végétaux indésirables par une quantité herbicide efficace d'un dérivé de cyclohexénone de formule I de la revendication 1.